# EUROPEAN PATENT SPECIFICATION

(11) **EP 1 127 143 B1**
(45) Date of publication and mention of the grant of the patent: **11.01.2006**
(21) Application number: 99955943.8
(22) Date of filing: 02.11.1999
(51) Int. Cl.: C12N 15/82, C07K 14/415, C12N 15/11, A01H 5/00

(54) **DNA COMPRISING RICE ANTHER-SPECIFIC GENE AND TRANSGENIC PLANT TRANSFORMED THEREWITH**
DNA MIT STAUBBEUTEL SPEZIFISCH EXPRIMIERTEM GEN AUS REIS UND DAMIT TRANSFORMIERTE TRANSGENE PFLANZE
GENE SPECIFIQUE POUR L'ANTHERE DU RIZ ET PLANTES TRANSGENIQUES TRANSFORMEES PAR CE GENE

(30) Priority: 03.11.1998 KR 9846973; 19.11.1998 KR 9850126
(43) Date of publication of application: 29.08.2001
(73) Proprietor: Syngenta Participations AG, 4058 Basel (CH)
(72) Inventor: AN, Gynheung, Pohang, Kyungbook 790-730 (KR); JEON, Jong-Seong, Davis, CA 95616 (US); CHUNG, Yong-Yoon, Daechi-dong, Seoul (KR); LEE, Sichul, Kangwon-do 235-900 (KR)
(74) Representative: Bastian, Werner Maria
(86) International application number: PCT/EP1999/008360
(87) International publication number: WO 2000/026389

(56) References cited:
- WO-A-89/10396
- WO-A-96/40925
- KOLTUNOW A M ET AL: "DIFFERENT TEMPORAL AND SPATIAL GENE EXPRESSION PATTERNS OCCUR DURING ANTHER DEVELOPMENT" PLANT CELL, vol. 2, no. 12, 1 December 1990 (1990-12-01), pages 1201-1224, XP002033709 ISSN: 1040-4651 cited in the application
- MATSUNGA S ET AL: "ISOLATION AND DEVELOPMENTAL EXPRESSION OF MALE REPRODUCTIVE ORGAN-SPECIFIC GENES IN A DIOECIOUS CAMPION, MELANDRIUM ALBUM (SILENE LATIFOLIA)" PLANT JOURNAL, vol. 10, no. 4, 1 January 1996 (1996-01-01), pages 679-689, XP000619376 ISSN: 0960-7412
- DATABASE EMEST26 [Online] EMBL Heidelberg, Germany AC/ID C72467, 19 September 1997 (1997-09-19) SASAKI T: "Rice cDNA from panicle at flowering stage" XP002135815
- JEON J-S ET AL.: "Isolation and characterization of an anther-specific gene, RA8, from rice (Oryza sativa L.)" PLANT MOLECULAR BIOLOGY, vol. 39, no. 1, January 1999 (1999-01), pages 35-44, XP002135814

### SEQUENCE LISTING

<110> Novartis AG
<120> Rice anther-specific gene
<130> S-30723A
<140>
   <141>
<150> KR 98-46973
   <151> 1998-11-03
<150> KR 98-50126
   <151> 1998-11-19
<160> 10
<170> PatentIn Ver. 2.2
<210> 1
   <211> 3003
   <212> DNA
   <213> Oryza sativa
<400> 1
   cattcagaat catctccagc ctacaatgta ctctctccca taatacaagt gtctctatga 60
   ttcaaaattt gtcctacaat ataaacattt ccagcatgaa atccatacat taattttcag 120
   ctaatcagat gcttggaggg aaaaatctaa gcgattcaat atgcaaaaat tgatcactga 180
   agtaactgaa agagaatatc tcgttttaac attagtgcta gtatttatta aacaactaaa 240
   aaattgttta tattttagta caaatcgagt agtagcagta gcagagctag cgtaagatcg 300
   tgttccgatc acctgagaaa ccgtcaggtg gtttgtctgt gccgtccagc cgatcagaat 360
   tcggagatcc gccgtcgttt ctttcctgaa atctgcaagt cccagcagca gcagcagcag 420
   agcaagagca atggcgtgca gggagtttga tactttgatg cactagctag ctactaggcg 480
   ttcgttccat gtcgctctca cgccgtgcga atgtgccatg atcctgcatg catcatcgcc 540
   aagattatat tcctcacatt ttttcttcct atcgctccta gtcgtctgtt tgggagctta 600
   aaattatgaa aagcagctgc tgagaagcta gctggtgaga atctgaagaa tttgagttct 660
   acgttcattc tccagattct acaattacag attcttataa tttaggtaaa aagctggact 720
   gtttgggagc ttctgtcagc cggagattct gtgagaagct gcagctgcta gaagcttccc 780
   caaacagacc cctagttgta ctctagctga tcgattcact ctattttata tacaccttgc 840
   tctctagctt atcaaacgta gccaagactt gaattttaaa gcttaaattg attttgatgt 900
   tcttttcatc gtaattcact taccgacctt agtcggcatt tgaattttta aaaataattt 960
   ttagagctga ttttgatttt tttttcagcg gaatttattt tcacgtatgt aaaagtttta 1020
   cctataaatt attaattttc agcggagtaa gcattagtgt tatgggttat aatcatctgg 1080
   tatgcttaaa tctctttact tggacttagt tgggacaatt cgtaatgcat tctcgtgcca 1140
   tctctataat acggcctgct agctttgctc ttgtatctgc acacaagaac tagctggcaa 1200
   agtcctcaag gcgaaccgcc tccatcttct ccttccagct cctcccatgg cgtccctcgt 1260
   cgccatcgcc atcgccatgg ctctcatggt gcagagatat ccagcatgct gaaattaact 1320
   tgacgcatat catctcatca tcacttgcat ttcaacttct ggattgtgca gacatgttta 1380
   gctgatcagt aaacgttgcc gtgttgaatt ggtccttatc aggtggttca acctggccgg 1440
   cagatgactg cctttgctgc tcggacgtcg ccggcggcgg cggcggaggc gttctggcgc 1500
   gccgccatgc ccggcgctcc catgccggac gccatcgtcg agctcctcca ccatggtgaa 1560
   tccgtgaact taattgtgta cactacttta aatgatacta gtattagtga tggtcactac 1620
   tagaaaacaa gtttttgcag gcagctagta aatatttcgc cggtaggcta aggcctgcga 1680
   aagtcgttga tttttgcagg cggagctcta tccgcttgcg cagacggcat ccttgtatca 1740
   cccacttgca aaaagttttt gggggcaaaa aagaaaaaga ttaaaaaaaa agcaatttcc 1800
   actacatgca aagcatccta ttcaaatcaa atcagattca ttcaacaaga aacacatcga 1860
   acagcagcta caatcagatg cactagaata aacactataa gctgacatgt aagatcctca 1920
   catatatgaa tgcttttgct gttatatatc tatttttttt aacgacggtt atatatctaa 1980
   ttaactaact atactataaa cttatctttt gttttttgct tttatctttt ataatatccg 2040
   ttgcaacgca cgaatcttta gctactgatc ataaaatgaa cagaaataag agattatgtt 2100
   tttatacgtg gagcctctct tacatgcgta gctgcatgcg tgcatgcaga gcacggcgtt 2160
   gctagcgccg gcggcaaggc caacggcggc ggcgacggtc caccgccgcc gatgaacttc 2220
   aactacgacg actacagggc cctgcctcgg agcgacgccc cttccccgga tgcattgaac 2280
   cgcgtcgccg ccgtacagaa cgccgacgag aacggcgtgt cctcgccgcc gccgccgccg 2340
   ccgacggtgt tcttcctcga ggacgccgtg cgcgtcggcg agagcctgcc cctaccgcga 2400
   ccggcggccg acgcaaccgc cgccggcgca gcggcggcca cggcgttgcc gccgctgcgg 2460
   ctgtacaccg tccggtcggt gagggcaatc gaggggtcca gcttcgtcgt gtgccgcggc 2520
   gagaccacgg cgggcgccgg cgtgtacggg tgccgccatg ccgccaccgg cccggcgagg 2580
   gcgtacgcgg tggacgcggc cggcggcggc ggcggggacg cggtgatcgc cgccgtcgtg 2640
   tgccacgccg acacgtcccg ctgggacccg gaccacgccg cgttccggct gctcggcgtc 2700
   aggcccggcg gcgccgccgt ctgccgcgcc gtcgccgacg cgcacatcct cccgacgaac 2760
   aaggattgag tacttctcgc catgatcgag gactcctcga tcgattagtg ctcgatcgat 2820
   cttaatctta ttgccatgca taattagtgt gttaattacg tgcttaattg tatgtgtgaa 2880
   gtgtgcatta gtcacagttt gtaatgtaat aatgtttcgc ttgttcgact gtatgatctc 2940
   gatttgggtg atatatacgt tgataaaata aaggaaaaag tttgtgttgg ttcatatgca 3000
   cga                                                               3003
<210> 2
   <211> 1436
   <212> DNA
   <213> Oryza sativa
<400> 2
   cattcagaat catctccagc ctacaatgta ctctctccca taatacaagt gtctctatga 60
   ttcaaaattt gtcctacaat ataaacattt ccagcatgaa atccatacat taattttcag 120
   ctaatcagat gcttggaggg aaaaatctaa gcgattcaat atgcaaaaat tgatcactga 180
   agtaactgaa agagaatatc tcgttttaac attagtgcta gtatttatta aacaactaaa 240
   aaattgttta tattttagta caaatcgagt agtagcagta gcagagctag cgtaagatcg 300
   tgttccgatc acctgagaaa ccgtcaggtg gtttgtctgt gccgtccagc cgatcagaat 360
   tcggagatcc gccgtcgttt ctttcctgaa atctgcaagt cccagcagca gcagcagcag 420
   agcaagagca atggcgtgca gggagtttga tactttgatg cactagctag ctactaggcg 480
   ttcgttccat gtcgctctca cgccgtgcga atgtgccatg atcctgcatg catcatcgcc 540
   aagattatat tcctcacatt ttttcttcct atcgctccta gtcgtctgtt tgggagctta 600
   aaattatgaa aagcagctgc tgagaagcta gctggtgaga atctgaagaa tttgagttct 660
   acgttcattc tccagattct acaattacag attcttataa tttaggtaaa aagctggact 720
   gtttgggagc ttctgtcagc cggagattct gtgagaagct gcagctgcta gaagcttccc 780
   caaacagacc cctagttgta ctctagctga tcgattcact ctattttata tacaccttgc 840
   tctctagctt atcaaacgta gccaagactt gaattttaaa gcttaaattg attttgatgt 900
   tcttttcatc gtaattcact taccgacctt agtcggcatt tgaattttta aaaataattt 960
   ttagagctga ttttgatttt tttttcagcg gaatttattt tcacgtatgt aaaagtttta 1020
   cctataaatt attaattttc agcggagtaa gcattagtgt tatgggttat aatcatctgg 1080
   tatgcttaaa tctctttact tggacttagt tgggacaatt cgtaatgcat tctcgtgcca 1140
   tctctataat acggcctgct agctttgctc ttgtatctgc acacaagaac tagctggcaa 1200
   agtcctcaag gcgaaccgcc tccatcttct ccttccagct cctcccatgg cgtccctcgt 1260
   cgccatcgcc atcgccatgg ctctcatggt gcagagatat ccagcatgct gaaattaact 1320
   tgacgcatat catctcatca tcacttgcat ttcaacttct ggattgtgca gacatgttta 1380
   gctgatcagt aaacgttgcc gtgttgaatt ggtccttatc aggtggttca acctgg 1436
<210> 3
   <211> 1196
   <212> DNA
   <213> Oryza sativa
<400> 3
   cattcagaat catctccagc ctacaatgta ctctctccca taatacaagt gtctctatga 60
   ttcaaaattt gtcctacaat ataaacattt ccagcatgaa atccatacat taattttcag 120
   ctaatcagat gcttggaggg aaaaatctaa gcgattcaat atgcaaaaat tgatcactga 180
   agtaactgaa agagaatatc tcgttttaac attagtgcta gtatttatta aacaactaaa 240
   aaattgttta tattttagta caaatcgagt agtagcagta gcagagctag cgtaagatcg 300
   tgttccgatc acctgagaaa ccgtcaggtg gtttgtctgt gccgtccagc cgatcagaat 360
   tcggagatcc gccgtcgttt ctttcctgaa atctgcaagt cccagcagca gcagcagcag 420
   agcaagagca atggcgtgca gggagtttga tactttgatg cactagctag ctactaggcg 480
   ttcgttccat gtcgctctca cgccgtgcga atgtgccatg atcctgcatg catcatcgcc 540
   aagattatat tcctcacatt ttttcttcct atcgctccta gtcgtctgtt tgggagctta 600
   aaattatgaa aagcagctgc tgagaagcta gctggtgaga atctgaagaa tttgagttct 660
   acgttcattc tccagattct acaattacag attcttataa tttaggtaaa aagctggact 720
   gtttgggagc ttctgtcagc cggagattct gtgagaagct gcagctgcta gaagcttccc 780
   caaacagacc cctagttgta ctctagctga tcgattcact ctattttata tacaccttgc 840
   tctctagctt atcaaacgta gccaagactt gaattttaaa gcttaaattg attttgatgt 900
   tcttttcatc gtaattcact taccgacctt agtcggcatt tgaattttta aaaataattt 960
   ttagagctga ttttgatttt tttttcagcg gaatttattt tcacgtatgt aaaagtttta 1020
   cctataaatt attaattttc agcggagtaa gcattagtgt tatgggttat aatcatctgg 1080
   tatgcttaaa tctctttact tggacttagt tgggacaatt cgtaatgcat tctcgtgcca 1140
   tctctataat acggcctgct agctttgctc ttgtatctgc acacaagaac tagctg     1196
<210> 4
   <211> 264
   <212> PRT
   <213> Oryza sativa
<400> 4
   Met Ala Ser Leu Val Ala Ile Ala Ile Ala Met Ala Leu Met Val Val
   1               5                   10                  15
   Gln Pro Gly Arg Gln Met Thr Ala Phe Ala Ala Arg Thr Ser Pro Ala
               20                  25                  30
   Ala Ala Ala Glu Ala Phe Trp Arg Ala Ala Met Pro Gly Ala Pro Met
           35                  40                  45
   Pro Asp Ala Ile Val Glu Leu Leu His His Glu His Gly Val Ala Ser
       50                  55                  60
   Ala Gly Gly Lys Ala Asn Gly Gly Gly Asp Gly Pro Pro Pro Pro Met
   65                  70                  75                  80
   Asn Phe Asn Tyr Asp Asp Tyr Arg Ala Leu Pro Arg Ser Asp Ala Pro
                   85                  90                  95
   Ser Pro Asp Ala Leu Asn Arg Val Ala Ala Val Gln Asn Ala Asp Glu
   100 105 110
   Asn Gly Val Ser Ser Pro Pro Pro Pro Pro Pro Thr Val Phe Phe Leu
   115 120 125
   Glu Asp Ala Val Arg Val Gly Glu Ser Leu Pro Leu Pro Arg Pro Ala
   130 135 140
   Ala Asp Ala Thr Ala Ala Gly Ala Ala Ala Ala Thr Ala Leu Pro Pro
   145 150 155 160
   Leu Arg Leu Tyr Thr Val Arg Ser Val Arg Ala Ile Glu Gly Ser Ser
   165 170 175
   Phe Val Val Cys Arg Gly Glu Thr Thr Ala Gly Ala Gly Val Tyr Gly
   180 185 190
   Cys Arg His Ala Ala Thr Gly Pro Ala Arg Ala Tyr Ala Val Asp Ala
   195 200 205
   Ala Gly Gly Gly Gly Gly Asp Ala Val Ile Ala Ala Val Val Cys His
   210 215 220
   Ala Asp Thr Ser Arg Trp Asp Pro Asp His Ala Ala Phe Arg Leu Leu
   225 230 235 240
   Gly Val Arg Pro Gly Gly Ala Ala Val Cys Arg Ala Val Ala Asp Ala
   245 250 255
   His Ile Leu Pro Thr Asn Lys Asp
   260
<210> 5
   <211> 15
   <212> DNA
   <213> Artificial Sequence
<220>
   <223> Description of Artificial Sequence: oligonucleotide
<400> 5
   ttggcatgca catac 15
<210> 6
   <211> 20
   <212> DNA
   <213> Artificial Sequence
<220>
   <223> Description of Artificial Sequence: oligonucleotide
<400> 6
   cgggatccgt gtttgacagg 20
<210> 7
   <211> 1008
   <212> DNA
   <213> Oryza sativa
<400> 7
   gcaaagtcct caaggcgaac cgcctccatc ttctccttcc agctcctccc atggcgtccc 60
   tcgtcgccat cgccatcgcc atggctctca tggtggttca acctggccgg cagatgactg 120
   cctttgctgc tcggacgtcg ccggcggcgg cggcggaggc gttctggcgc gccgccatgc 180
   ccggcgctcc catgccggac gccatcgtcg agctcctcca ccatgagcac ggcgttgcta 240
   gcgccggcgg caaggccaac ggcggcggcg acggtccacc gccgccgatg aacttcaact 300
   acgacgacta cagggccctg cctcggagcg acgccccttc cccggatgca ttgaaccgcg 360
   tcgccgccgt acagaacgcc gacgagaacg gcgtgtcctc gccgccgccg ccgccgccga 420
   cggtgttctt cctcgaggac gccgtgcgcg tcggcgagag cctgccccta ccgcgaccgg 480
   cggccgacgc aaccgccgcc ggcgcagcgg cggccacggc gttgccgccg ctgcggctgt 540
   acaccgtccg gtcggtgagg gcaatcgagg ggtccagctt cgtcgtgtgc cgcggcgaga 600
   ccacggcggg cgccggcgtg tacgggtgcc gccatgccgc caccggcccg gcgagggcgt 660
   acgcggtgga cgcggccggc ggcggcggcg gggacgcggt gatcgccgcc gtcgtgtgcc 720
   acgccgacac gtcccgctgg gacccggacc acgccgcgtt ccggctgctc ggcgtcaggc 780
   ccggcggcgc cgccgtctgc cgcgccgtcg ccgacgcgca catcctcccg acgaacaagg 840
   attgagtact tctcgccatg atcgaggact cctcgatcga ttagtgctcg atcgatctta 900
   atcttattgc catgcataat tagtgtgtta attacgtgct taattgtatg tgtgaagtgt 960
   gcattagtca cagtttgtaa tgtaataatg tttcgcttgt tcgactgt 1008
<210> 8
   <211> 20
   <212> DNA
   <213> Artificial Sequence
<220>
   <223> Description of Artificial Sequence: oligonucleotide
<400> 8
   aattaaccct cactaaaggg 20
<210> 9
   <211> 20
   <212> DNA
   <213> Artificial Sequence
<220>
   <223> Description of Artificial Sequence: oligonucleotide
<400> 9
   gcggatccag gttgaaccac 20
<210> 10
   <211> 240
   <212> DNA
   <213> Oryza sativa
<400> 10
   gcaaagtcct caaggcgaac cgcctccatc ttctccttcc agctcctccc atggcgtccc 60
   tcgtcgccat cgccatcgcc atggctctca tggtgcagag atatccagca tgctgaaatt 120
   aacttgacgc atatcatctc atcatcactt gcatttcaac ttctggattg tgcagacatg 180
   tttagctgat cagtaaacgt tgccgtgttg aattggtcct tatcaggtgg ttcaacctgg 240

## Description

The present invention is in the field of plant genetic engineering.
It primarily relates to novel DNA sequences which function as promoters of anther-specific transcription of coding DNA sequences in recombinant or chimeric DNA sequences. The present invention also relates to recombinant or chimeric DNA sequences, which are expressed specifically in the anther of a plant. The said recombinant or chimeric DNA sequences may be used to create transgenic plants, but especially transgenic male-sterile plants.

The creation of male sterile plants is of economic interest in the production of hybrid seeds. Male sterility prevents self-pollination which otherwise occurs in many plant species and hinders breeding and hybrid seed production. The anther is a male-reproductive organ of flowering plants, which is composed of several tissues, such as, tapetum, endothecium, connective tissues, vascular tissues, etc. and is responsible for producing pollen. Anther development can be divided into two general phases. During phase 1, the morphology of the anther is established and microspore mother cells undergo meiosis to generate tetrads of microspores. During phase 2, pollen grains and anthers differentiate and tissue degeneration, dehiscence, and pollen grain release occur. Among many diverse genes involved in these developments, only a small fraction is anther-specific.
Anther-specific genes, which have been reported hitherto, include Osc4, Osc6, YY1 and YY2 genes of rice [Hihara Y, et al., Plant Mol. Biol., 30:1181-1193(1996); Tsuchiya T., et al., Plant Mol. Biol., 26:1737-1747(1994)], TA29 and TA32 genes of tobacco [Koltunow, A.M. et al., Plant Cell, 2:1201-1224(1990)], SF2 and SF18 genes of sunflower [Domon, C. et al., Plant Mol. Biol., 15: 643-646(1990)], 108 genes of tomato [Smith, A.G., et al., Mol. Gen. Genet., 222:9-16(1990)], NTM 19 of tobacco [Oldenhof M.T. et al. Plant Mol Biol 31:213-225 (1996)], and BA42, BA112 and A9 genes of Brassica napus [Scott, R. et al., Plant Mol. Biol., 17:195-207(1991); Shen, J.B. et al., Mol. Gen. Genet., 234:379-389(1992)].
Some of these genes are found exclusively in sporophytic tissues of the anthers, others are pollen grain specific or are present in both, sporophytic and gametophytic tissues of the anthers.
Rice is an example for a plant which propagates in self-pollination fashion. It is difficult to develop hybrid rice only by allogamy. To facilitate the production of hybrid rice by allogamy, a method is described in the prior art, wherein the anthers are deleted from rice flowers to produce male-sterile rice, and then pollen from another rice plant is transferred thereto. However, there is the problem that such a method requires a large amount of time and effort.
Thus, in order to produce rice or any other self-pollinating plant having a male-sterile character without the prior problems as described above, the present inventors increasingly made an effort to develop a method using a gene which induces an abnormal development of the anther.

The invention thus provides: a DNA molecule according to claim 1. Also disclosed are DNA molecules and methods for the specific expression of a coding region of interest In the tapetum, endothecium and connective tissues of anthers but not in microspores or pollen, wherein expression of the coding sequence of interest starts at the tetrad stage and reaches a maximum level at the vacuolated pollen stage. In particular, DNA molecules and methods are provided wherein
- the DNA comprises a nucleotide sequence having at least 50%, particularly 65% more particularly 80%, and most particularly 90% or more sequence identity with the sequence shown In SEQ ID NO:1
- the DNA according to the invention comprises the nucleotide sequence shown in SEQ ID NO:1
- the DNA molecules are specifically designed such that the coding sequence is in antisense orientation

The invention further provides DNA molecules and methods wherein the coding sequence encodes a polypeptide which will disrupt formation of viable pollen when expressed in the anther calls. In a specific embodiment of the invention, the coding sequence encodes a polypeptide selected from the group consisting of RNase, DTA, TURF-13, pectate lyase, gin recombinase, iaaL and cytA toxin.

Further disclosed are DNA molecules and methods as mentioned before, wherein the DNA sequence of the invention has more than 80%, particularly 90% and more particularly 95% sequence identity in the consecutive 30 bases of any sites of SEQ ID NO:1.
The invention further provides DNA molecules comprising a promoter sequence capable of driving expression of an associated coding sequence specifically in the tapetum, endothecium and connective tissue of anthers but not in microspores or pollen, wherein expression of the coding sequence starts at the tetrad stage and reaches a maximum level at the vacuolated pollen stage.

In particular, we provide herein methods and DNA molecules as mentioned before, wherein
- the promoter sequence has 50% particularly 65% more particularly 80%, and most particularly 90% or more sequence identity with the nucleotide sequence of SEQ ID NO:3
- the promoter sequence has the nucleotide sequence shown in SEQ ID NO:3
- the promoter sequence comprises an additional sequence that can be operatively linked to a coding sequence of interest
- the additional sequence comprises a sequence having 50%, particularly 65%, more particularly 80%, and most particularly 90% or more sequence identity with SEQ ID NO:10
- the additional sequence comprises SEQ ID NO:10
- the promoter sequence and additional sequence have 50%, particularly 65%, more particularly 80%, and most particularly 90% or more sequence identity with SEQ ID NO:2
- the promoter sequence and additional sequence are characterized by the nucleotide sequence of SEQ ID NO:2.

In addition, DNA molecules are provided wherein the promoter comprises a fragment obtainable from SEQ ID NO:2, preferably a fragment capable of driving expression of an associated coding sequence specifically in the tapetum, endotheclum and connective tissue of anthers but not in microspores or pollen, wherein expression of the coding sequence starts at the tetrad stage and reaches a maximum level at the vacuolated pollen stage. Also disclosed are DNA molecules comprising an open reading frame encoding a protein characterized by an amino acid sequence having 50%, particularly 65%, more particularly 80%, and most particularly 90% or more sequence identity with SEQ ID NO:4. In a specific embodiment of the invention, the open reading frame encodes a protein characterized by the amino acid sequence of SEQ ID NO:4. Further disclosed is a DNA molecule characterized by SEQ ID NO:7.
In addition are provided expression vectors comprising a first expression cassette comprising a DNA of the invention for expression in a host organism such as a microorganism or a plant and, optionally, a second expression cassette comprising a gene of interest. In a specific embodiment of the invention, the second expression cassette comprises a marker gene.
The invention further provides the protein encoded by the open reading frame as mentioned hereinbefore.
In addition, transgenic plants and plant material and the sexual and/or asexual progeny thereof are provided, which have been transformed with a DNA sequence according to the invention. In particular the invention provides
- a transgenic, male-sterile plant which has been transformed with a DNA sequence of the invention
- a transgenic plant or plant material according to the invention, wherein the plant is rice, wheat, maize, *Sorghum bicolor* or orchardgrass
The invention further provides a process for the production of a transgenic plant comprising a DNA comprising a promoter sequence and associated coding sequence wherein the promoter sequence drives expression of the coding sequence specifically in the tapetum, endothecium and connective tissues of anthers but not in microspores or pollen, and wherein expression of the coding sequence starts at the tetrad stage and reaches a maximum level at the vacuolated pollen stage. In particular, a process wherein the plant is rice, wheat, maize, *Sorghum bicolor* or orchardgrass.

### The constitution and function of the invention

Also disclosed is a rice (*Oryza sativa* L.) anther-specific gene having the base sequence shown in SEQ ID NO:1 and a gene having more than 80% sequence identity in the consecutive 30 bases of any sites on said gene. In addition, the present invention provides the anther-specific expression regulator comprising a promoter sequence and additional sequence having SEQ ID NO:2 corresponding to the base sequences between -1196 and 240 in said gene, represented by nt 1 to nt 1436 of SEQ ID NO:1.
The present invention also provides the anther specific expression promoter having SEQ ID NO:3, corresponding to the base sequences between the -1196 and -1 in said gene, represented by nt 1 to nt 1196 of SEQ ID NO:1.

### DEFINITIONS

In order to ensure a clear and consistent understanding of the specification and the claims, the following definitions are provided:
Chimeric: is used to indicate that a DNA sequence, such as a vector or a gene, is comprised of more than one DNA sequences of distinct origin which are fused together by recombinant DNA techniques resulting in a DNA sequence, which does not occur naturally, and which particularly does not occur in the plant to be transformed
Expression: refers to the transcription and/or translation of an endogenous gene or a transgene in plants. In the case of antisense constructs, for example, expression may refer to the transcription of the antisense DNA only
Gene: refers to a coding sequence and associated regulatory sequence wherein the coding sequence is transcribed into RNA such as mRNA, rRNA, tRNA, snRNA, sense RNA or antisense RNA. Examples of regulatory sequences are promoter sequences, 5' and 3' untranslated sequences and termination sequences. Further elements that may be present are, for example, introns
Marker gene: refers to a gene encoding a selectable or screenable trait
nt abbreviation for 'nucleotide'
Operatively linked to/associated with: a regulatory DNA sequence is said to be "operatively linked to" or "associated with" a DNA sequence that codes for an RNA or a protein if the two sequences are situated such that the regulatory DNA sequence affects expression of the coding DNA sequence
Plant: refers to any plant, particularly to seed plants
Plant material: refers to leaves, stems, roots, flowers or flower parts, fruits, pollen, pollen tubes, ovules, embryo sacs, egg cells, zygotes, embryos, seeds, cuttings, cell or tissue cultures, or any other part or product of a plant
Promoter: refers to a DNA sequence that initiates transcription of an associated DNA sequence. The promoter region may also include elements that act as regulators of gene expression such as activators, enhancers, and/or repressors
Recombinant DNA molecule: a combination of DNA sequences that are joined together using recombinant DNA technology
Recombinant DNA technology: procedures used to join together DNA sequences as described, for example, in Sambrook et al., 1989, Cold Spring Harbor, NY: Cold Spring Harbor Laboratory Press
Screenable marker gene: refers to a gene whose expression does not confer a selective advantage to a transformed cell, but whose expression makes the transformed cell phenotypically distinct from untransformed cells
Selectable marker gene: refers to a gene whose expression in a plant cell gives the cell a selective advantage. The selective advantage possessed by the cells transformed with the selectable marker gene may be due to their ability to grow in the presence of a negative selective agent, such as an antibiotic or a herbicide, compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell gives the cell both, a negative and a positive selective advantage
Sequence identity: the percentage of sequence identity is determined using computer programs that are based on dynamic programming algorithms. Computer programs that are preferred within the scope of the present invention include the BLAST (Basic Local Alignment Search Tool) search programs designed to explore all of the available sequence databases regardless of whether the query is protein or DNA. Version BLAST 2.0 (Gapped BLAST) of this search tool has been made publicly available on the Internet (currently http://www.ncbi.nlm.nih.gov/BLAST/). It uses a heuristic algorithm, which seeks local as opposed to global alignments and is therefore able to detect relationships among sequences, which share only isolated regions. The scores assigned in a BLAST search have a well-defined statistical interpretation. Said programs are preferably run with optional parameters set to the default values.
Transformation: refers to the introduction of a nucleic acid into a cell. In particular, it refers to the stable integration of a DNA molecule into the genome of an organism of interest.

### BRIEF DESCRIPTION OF THE SEQUENCES IN THE SEQUENCE LISTING

- SEQ ID NO:1: nucleotide sequence of the rice RA8 gene
- SEQ ID NO:2: anther-specific expression regulator comprising the RA8 promoter plus first exon, first intron and part of exon 2
- SEQ ID NO:3: RA8 promoter
- SEQ ID NO:4: deduced amino acid sequence of the protein encoded by the rice RA8 gene
- SEQ ID NO:5: primer 1
- SEQ ID NO:6: primer 2
- SEQ ID NO:7: RA8 cDNA sequence
- SEQ ID NO:8: PCR primer
- SEQ ID NO:9: PCR primer
- SEQ ID NO:10: nucleotide sequence of the first exon, first intron and part of exon 2 of the rice RA8 gene

### DEPOSITS

| **Deposited material** | **Accession number** | **Date of deposit** |
|---|---|---|
| plasmid pGA1173-9 | KCTC 8899P | July 29, 1998 |

The deposit is made with the Korea Research Institute of Bioscience and Biotechnology, the annex organization of Korea Advanced Institute of Science and Technology (KAIST).

Hereinafter, the present invention will be described In detail.
The gene of the present disclosure is isolated from rice (*Oryza sativa* L), expresses specifically in the anther, and the genomic sequence thereof is represented in SEQ ID NO:1. The cDNA sequence of the RA8 gene is shown in SEQ ID NO:7. Comparison of the cDNA and genomic sequences reveals the presence of 2 introns and 3 exons In the coding sequence of the RA8 gene. Intron 1 of 134 bp is located between the 14th and 15th codons, corresponding to nt 1289 to nt 1422 of SEO ID NO:1, and corresponding to nt 1556 to nt 2149 of SEQ ID NO:1, and intron 2 of 594 bp is located at the 59th codon in the amino acid coding region which is deduced from the said base sequences, corresponding to nt 1556 to nt 2149 of SEQ ID NO:1. Both introns contain the consensus GT and AG sequences at the 5' and 3' ends, respectively. With respect to SEQ ID NO:1, the three exons are located as follows: exon 1: nt 1247 to nt 1288; exon 2: nt 1423 to 1555; exon 3: nt 2150 to nt 2766. In the 5' non-coding flanking sequence of said coding region, a CAAT box sequence, CAAT, is located at base position -82 to -79, corresponding to nt 1116 to nt 1119 of SEQ ID NO:1, and a TATA box sequence, TATAATA, is located at base position -53 to -47, corresponding to nt 1145 to nt 1151 of SEQ ID NO:1. The poly (A) tail is located at the 164th base (nucleotide 2932 of SEQ ID NO:1) downstream from the TGA translation termination codon. In the 3' non-coding region, the AATAA consensus polyadenylation signal sequence is present. The sequence surrounding the first ATG fits well with the translation initiating consensus sequence of monocots as reported in the literature [Joshi, C.P. et al., Plant Mol. Biol., 35:993-1001 (1997)].

An open reading frame of said coding region consists of 264 amino acid residues and has an amino acid sequence of SEQ ID NO:4.
A protein deduced from said open reading frame, has molecular mass of 26.4 kDa and a pl of 6.1. Major amino acids constituting the protein are alanine (21.9%), glycine (9.9%) and proline (10.2%), and the amino acid sequence thereof contains a hydrophobic N-terminal region which may be involved in targeting the protein into a membrane fraction or extra-cellular space, an extensin-like SPPPPPP motif and a glycine-rich region. According to a homology analysis with Genebank databases, such amino acid sequence is novel, and does not show a significant sequence identity to any genes as reported hitherto.

The expression of the gene according to the present invention is characterized by being regulated temporally and spatially. The spatial expression pattern includes that the gene is expressed only in the anther organ of rice flower, but not in other floral organs other than the anther, leaves and roots, etc., and especially among the anther organ, it is expressed only in tapetum, endothecium, and connective tissues, but not in vascular tissues. The temporal expression pattern includes that the expression levels of the gene increase as flowers mature, wherein the gene is scarcely expressed at pre-meiosis stage, but the expression thereof is first detectable at the time when microspores are released from tetrads. The maximum levels are observed at the vacuolated-pollen stage, and sharply decrease at the mature pollen stage immediately prior to blooming.

The gene of the present invention can be obtained by means of differential hybridization methods. For example, by a method which comprises hybridizing an anther cDNA library with a cDNA library of non-anther origin, such as from leaves, roots or seedlings, and thereafter obtaining the cDNA clone of an anther-specific gene which negatively reacts with the cDNA library of non-anther origin. In addition, the obtained cDNA clone is used as a probe to hybridize it with a genomic library, and isolate a clone which positively reacts therewith, and to subclone it to obtain a genomic clone of the anther-specific gene. Another method for obtaining the gene includes a method, wherein the base sequences of said SEQ ID NO:1 are used to synthesize an appropriate primer for a conventional PCR method. In a specific embodiment of the invention, the obtained gene is cloned into a pBluescript SK (-) vector and designated as plasmid pGA1173-9. This plasmid is deposited at the Korea Research Institute of Bioscience and Biotechnology, the annex organization of Korea Advanced Institute of Science and Technology (KAIST) under the deposition No. KCTC 8899P on July 29, 1998.

The region containing the RA8 promoter is shown in SEQ ID NO:3, corresponding to nt 1 to nt 1196 of SEQ ID NO:1. The transcribed nucleotides of the RA8 gene start at position 1197 of SEQ ID NO:1. An element which regulates the anther-specific expression of the gene of the present invention is located in the region containing the promoter, exon 1, intron 1 and part of exon 2, and such region can be represented as SEQ ID NO:2 corresponding to the base sequence between nt 1 and nt 1436 in the base sequence of said SEQ ID NO:1. This regulatory element can be obtained by a conventional cloning method from the rice genomic DNA or plasmid pGA1173-9, for example, wherein the base sequence of said SEQ ID NO:2 is used to synthesize the appropriate primer, and thereafter a conventional PCR method is performed.

The plant expression vector of the present invention contains for example the said regulatory element as shown in SEQ ID NO:2, wherein the anther-specific expression regulator is fused with another gene of interest. As the desired gene, a reporter gene, such as beta-glucuronidase (GUS) may be used, and for the purpose of inducing abnormal development of rice anthers, DTA (a toxic gene which disrupts cells), RNase gene, etc. may be used (see below) but the gene of interest should not be limited thereto.
The plant expression vector may contain a second expression cassette comprising a marker gene to allow selection of transformants. Examples of selectable or screenable marker genes are described below. For certain target species, different antibiotic or herbicide selection markers may be preferred. Selection markers used routinely in transformation include the *npt*II gene which confers resistance to kanamycin, paromomycin, geneticin and related antibiotics (Vieira and Messing, 1982, Gene 19: 259-268; Bevan et al., 1983, Nature 304:184-187) the bacterial aadA gene (Goldschmidt-Clermont, 1991, Nucl. Acids Res. 19: 4083-4089), encoding aminoglycoside 3'-adenylyltransferase and conferring resistance to streptomycin or spectinomycin, the *hph* gene which confers resistance to the antibiotic hygromycin (Biochlinger and Diggelmann, 1984, Mol. Cell. Biol. 4: 2929-2931), and the *dhfr* gene, which confers resistance to methotrexate (Bourouis and Jarry, 1983, EMBO J. 2: 1099-1104). Other markers to be used include a phosphinothricin acetyltransferase gene, which confers resistance to the herbicide phosphinothricin (White et al., 1990, Nud. Adds Res. 18: 1062; Spencer et al. 1990, Theor. Appl. Genet. 79: 625-631), a mutant EPSP synthase gene encoding glyphosate resistance (Hinchee et al., 1988, Bio/Technology 6: 915-922), a mutant acetolactate synthase (ALS) gene which confers imidazolione or sulfonylurea resistance (Lee et al., 1988, EMBO J. 7: 1241-1248), a mutant psbA gene conferring resistance to atrazine (Smeda et al., 1993, Plant Physiol. 103: 911-917), or a mutant protoporphyrinogen oxidase gene as described in EP 0 769 059. Selection markers resulting in positive selection, such as a phosphomannose isomerase gene, as described in patent application WO 93/05163, are also used.
Identification of transformed cells may also be accomplished through expression of screenable marker genes such as genes coding for chloramphanicol acetyl transferase (CAT), β-glucuronidase (GUS), luciferase, and green fluorescent protein (GFP) or any other protein that confers a phenotypically distinct trait to the transformed cell.

The said expression vector may be constructed by fusing the said anther-specific expression regulator with the desired gene, and thereafter ligating it to an appropriate plant expression vector. For example, the regulatory element is fused with GUS without changes of reading frame, and the fused product is then ligated to a binary vector pGA1633 capable of expressing in plants to construct an expression vector pGA1647.

The invention also provides a process according to claim 14.

The recombinant DNA sequences of the invention can be introduced into the plant cell by a number of well-known methods. Those skilled In the art will appreciate that the choice of such method might depend on the type of plant which is targeted for transformation, i.e., monocot or dicot. Suitable methods of transforming plant cells include microinjection (Crossway et al., 1986, Bio Techniques 4:320-334), electroporation (Riggs and Bates, 1986, Proc. Natl. Acad. Sci., USA 83:5602-5606), Agrobacterium-mediated transformation (Hinchee et al., 1988, Bio/Technology 6:915-922; EP 0 853 675), direct gene transfer (Paszkowski et al., 1984, EMBO J. 3:2717-2722), and ballistic particle acceleration using devices available from Agracetus, Inc., Madison, Wisconsin and Dupont, Inc., Wilmington, Delaware(see, for example, US Patent No. 4,945,050 and McCabe et al., 1988, Bio/Technology 6:923-926). The cells to be transformed may be differentiated leaf cells, embryogenic cells, or any other type of cell.
In the direct transformation of protoplasts, the uptake of exogenous genetic material into a protoplast may be enhanced by the use of a chemical agent or an electric field. The exogenous material may then be integrated into the nuclear genome. The previous work is conducted in dicot tobacco plants, which resulted in the foreign DNA being incorporated and transferred to progeny plants (Paszkowski et al., 1984, EMBO J. 3:2712-2722; Potrykus et al., 1985, Mol. Gen. Genet 199:169-177). Monocot protoplasts, for example, of Triticum monococcum, Lolium multiflorum(Italian rye grass), maize, and Black Mexican sweet corn, are transformed by this procedure. An additional preferred embodiment is the protoplast transformation method for maize as disclosed in EP 0 292 435, as well as in EP 0 846 771. For maize transformation also see Koziel et al., 1993, Bio/Technology 11:194-200. Transformation of rice can be carried out by direct gene transfer techniques utilizing protoplasts or particle bombardment. Protoplast-mediated transformation is described for Japonica-types and Indica-types (Zhang et al., 1988, Plant Cell Rep., 7:379-384; Shimamoto et al., 1989, Nature 338:274-276; Datta et al., 1990, Bio/Technology 8:736-740). Above both types are also routinely transformable using the particle bombardment (Christou et al., 1991, Bio/Technology 9:957-962). Patent application No. EP 0 332 581 describes techniques for the generation, transformation and regeneration of Pooideae protoplasts. These techniques allow the transformation of all Pooideae plants including Dactylis and wheat. Furthermore, wheat transformation is described in patent application No. EP 0 674 715; and by Weeks et al., 1993 (Plant Physiol. 102:1077-1084).
The thus-constructed plant expression vector can, for example, be introduced into the calli of rice according to the conventional plant transformation method, and the differentiation of roots and leaves is induced therefrom, and then, can be transferred to a flowerpot for cultivation, thereby obtaining the transformed rice.
The preferred method for transforming plants is the A*grobacterium*-mediated transformation. According to the Agrobacterium-mediated transformation as a representative example, the expression vector PGA1647 is transferred to the *Agrobacterium tumefaciens* carrying the binary Ti plasmid using the freeze-thaw method (An, G. et al., (eds) Plant Molecular Biology Manual, pp. A3/1-A3/19, Kluwer Academic Publishers, Dordrecht (1998)), and then grown in an AB liquid medium supplemented with the appropriate antibiotics (Chilton, M-D, Pro. Natl. Acad. Sci. USA, 71, 3672-3676 (1974)), and the thus-obtained product is co-cultivated with calli on a 2N6-As medium supplemented with 1 mM betaine (Hiei, Y. et al., Plant J., 6, 271-282 (1994)) in darkness at 25°C for 2-3 days. The co-cultivated calli are washed with sterile water containing cefotaxime, and again incubated on an N6 medium containing an appropriate antibiotic and cefotaxime for 3-4 weeks to obtain actively growing calli. These calli are transferred onto an appropriate selection medium, for example, MS media (Life Technologies) supplemented with 0.2 mg/L NAA (naphthalene acetic acid), 2 mg/L kinetin, 2% sorbitol, 1.6% phytagar (Gibco), the appropriate antibiotic, and 250 mg/L cefotaxime, and then cultivated for 2-3 weeks under continuous light condition of 30-60 µmol m⁻² sec⁻¹, preferably 40 µmol m⁻² sec⁻¹ to obtain plantlets. These plantlets are potted and grown in a growth chamber under the condition of 10h light/14h dark to obtain the transgenic rice.

In case that the vector used in the said method contains the fused gene of the regulatory element of the present invention and DTA or RNase genes etc., the plant transformed with the vector has the male-sterile character.

The coding DNA sequence of the present invention may be any DNA sequence encoding a desired polypeptide. Especially preferred for use in the present invention are, however, coding DNA sequences which encode a product of polypeptide which, when expressed in anther tissue, make the plant not produce viable pollen. Examples of such coding DNA sequences include the genes which are described in the following reference:
a) Diptheria toxin A-chain gene (DTA), which inhibits protein synthesis (Greenfield et al., 1983, Proc. Natl. Acad. Sci., USA, 80:6853; Palmiter et al., 1987, Cell 50:435-443);
b) Pectate lyase gene peIE from Erwinia chrysanthemi EC16, which degrades pectin, causing cell lysis (Keen et al., 1986, J. Bacteriology 168:595);
c) T-urf13 (TURF-13) gene from cms-T maize mitochondrial genomes: this gene encodes a polypeptide designated as URF13 which disrupts mitochondrial or plasma membranes (Braun et al., 1990, Plant Cell 2: 153; Dewey et al., 1987, Proc. Natl. Acad. Sci., USA, 84:5374 and Dewey et al., 1986, Cell 44:439);
d) Gin recombinase gene from phage Mu gene, which encodes a site-specific DNA recombinase which will cause genome rearrangements and loss of cell viability when expressed in plant cells (Maeser et al., 1991, Mol Gen Genet 230:170-176);
e) Indole acetic acid-lysine synthetase gene (iaaL) from Pseudomonas syringae, which encodes an enzyme that conjugates lysine to indoleacetic acid (IAA). When expressed in the cells of plants, it causes an altered development due to the removal of IAA from the cell via conjugation (Romano et al., 1991, Genes and Development 5:438-446; Spena et al., 1991, Mol Gen Genet 227:205-212; Roberto et al., 1990, Pro Natl Acad Sci USA, 87:5795-5801); and
f) CytA toxin gene from Bacillus thuringiensis israeliensis which encodes a mosquitocidal and hemolytic protein. When expressed in plant cells, it causes death of the cell due to disruption of the cell membrane (McLean et al., 1987, J. Bacteriology 169:1017-1023; Ellar et al., US Patent No. 4,918,006, 1990).

### EXAMPLES

The present invention will now be described in more detail by reference to the Examples, but it should not be construed as a limitation upon the scope of the present invention.

### Example 1: Isolation of rice anther-specific gene, RA8

### Step 1: Isolation of the RA8 gene

Rice (*Oryza sativa* L. Nakdong) flowers at the late-vacuolated stage are cross-sectioned into three parts- The middle portion, which contains intact anthers and parts of the palea and lemma, is used as an anther-enriched sample. From this portion, anther-enriched mRNA is isolated using poly (A) Quick mRNA Isolation Kit (Stratagene, USA). The anther cDNA libraries are constructed with ZAP-cDNA Gigapack 2 Gold Cloning Kit (Stratagene) which uses the mRNA as a template.
The same process is applied on leaves, which are harvested from six-day-old seedlings of rice, to construct leaf cDNA libraries.
From the constructed anther cDNA libraries, 33 cDNA clones are randomly selected and thereafter, cDNA regions, which are obtained by cleaving the respective clone DNA with EcoRI, are labeled with a radioactive isotope 32P and hybridized with plaques from the leaf cDNA library.
As a result of this, six anther cDNA clones do not positively react with the plaques from the leaf cDNA library, and among these clones, one clone, which is most abundantly present in the anther cDNA library, is selected and designated as RA8.
From this RA8 clone, the f1 helper phage R408 (Stratagene) is used to obtain a recombinant plasmid pGA1173-4 (RA8), into which cDNA fragment is inserted by in vivo excision, and the base sequences of RA8 cDNA, which are cloned into the above plasmid, are determined by the method of Sanger et al. (Sanger, F. et al., Proc. Natl. Acad. Sci. USA, 74, 5463-5467(1977)).
The said base sequences are represented by SEQ ID NO:7. As shown in SEQ ID NO:7, the base sequence of the RA8 cDNA is 1008 bps in size. An open reading frame is present from nt 51 to nt 842 of SEQ ID NO:7 consisting of 264 amino acid residues. The protein deduced from said open reading frame has molecular mass of 26.4 kDa and a pl of 6.1. The sequence surrounding the first ATG fits well with the translational start consensus sequence of monocots as reported in the literature [Joshi, C.P. et al., Plant Mol. Biol., 35:993-1001(1997)], the poly (A) tail is located at the 164th base sequence downstream from the TGA translation termination codon. In the 3' non-coding region, AATAA consensus polyadenylation signal sequence is present.
Major amino acid residues constituting the above amino acid sequence are alanine (21.9%), glycine (9.9%) and proline (10.2%), and the amino acid sequence contains a hydrophobic N-terminal region which may be involved in targeting the protein into a membrane fraction or extra-cellular space, an extensin-like SPPPPPP motif and glycine-rich region. According to a homology analysis with Genebank databases, it is confirmed that such amino acid sequence is a novel gene, which does not show a significant sequence identity to any genes as reported hitherto.

### Step 2: RNA blotting analysis

To confirm whether the RA8 gene obtained from the above step 1, is expressed specifically in the anthers, total RNA is isolated from leaves, roots, young flowers, mature flowers, and paleas/lemmas, pistils and stamens of flowers of rice, electrophoresed, and then transferred to a nylon membrane. The 32P-labeled RA8 cDNA is used as a probe to perform the RNA blotting.
The RA8 mRNA is present only in flowers at various developmental stages but not in leaves and roots. The expression level of the RA8 gene increases during flower development, reaching the highest level in mature flowers. In order to determine the organ specificity within a flower, an RNA blot experiment is conducted with the total RNA isolated from different floral organs. Each of the floral organs is dissected under a dissecting microscope to minimize cross-contamination. The result revealed that the RA8 mRNA is present in anthers, but not in the carpel or the palea/lemma.

### Step 3: Isolation of the RA8 genomic clone

Plaques of rice genomic libraries (which are constructed by standard methods and is obtained from Steve Kay and Nam-Hi Chua of Rockefeller University in the form of EMBL lambda phage DNA, into which the rice genomic DNA is inserted) are transferred to a nylon membrane (Hybond, Amersham), and hybridized by using the radioactive isotope 32P-labeled RA8 cDNA as a hybridization probes. The hybridized nylon membrane is exposed to X-ray film, and phage clones are obtained from plaques which is positively shown.
The lambda phage DNA is isolated from these phage clones, cleaved with BamHI to obtain a 13.7 kb genomic fragment, and among this a 4.3 kb SacI-EcoRI fragment is sub-cloned. The 2.9 kb SacI fragment obtained from the 4.3 kb SacI-EcoRI fragment which contains the 5' flanking sequence and the 5' coding region, whereas the adjacent 1.5 kb SacI-EcoRI fragment contains the remaining coding region as well as the 3' flanking sequence. Two introns (intron 1 and intron 2) and 3 exons are present in the coding region. With respect to SEQ ID NO:1, the three exons are located as follows: exon 1 : nt 1247 to nt 1288; exon 2: nt 1423 to 1555; exon 3: nt 2150 to nt 2766. Intron 1 is 134 bps in size and located between the 14th and 15th codons, corresponding to nt 1289 to nt 1422 of SEQ ID NO:1, and intron 2 is 594 bps in size and located at the 59th codon, corresponding to nt 1556 to nt 2149 of SEQ ID NO:1. Both introns contain the consensus GT and AT sequences at the 5' and 3' ends, respectively. The 5' flanking sequence of the RA8 coding region contains CAAT box sequence, CAAT at the base position -82 to -79, corresponding to nt 1116 to nt 1119 of SEQ ID NO:1, and TATA box sequence, TATAATA at the base position -53 to -47, corresponding to nt 1145 to nt 1151 of SEQ ID NO:1.
The 2.9 kb SacI fragment is cloned into the plasmid pBluescript SK (-) vector, and this plasmid is designated as pGA1173-9. This plasmid is deposited at Korea Research Institute of Bioscience and Biotechnology, the annex organization of Korea Advanced Institute of Science and Technology (KAIST) under deposition No. KCTC 8899P on July 29, 1998.
Base sequences of genomic DNA fragment, which are cloned into the plasmid pGA1173-9, are analyzed by using the method of Sanger et al, and an approximate 2.5 kb promoter region is determined in comparison with cDNA.
To study genomic complexity of the RA8 clone, the genomic DNA isolated from leaves of two-week-old rice seedlings is cleaved with EcoRI, HindIII or Pstl, and then subjected to DNA-blotting with the RA8 cDNA. The RA8 cDNA probe hybridized with a single band of the rice genomic DNA, and thus the RA8 gene exists as a single copy in the rice genome.

### Example 2: Production of plant expression vector

The binary vector, which contains a reporter gene, GUS and a selection gene, hph (hygromycin phosphotransferase), is constructed as follows.
A plasmid pGA748 (An, G. Binary Ti plasmid vectors, Agrobacterium protocols, Humana Press. pp. 47-58) is cleaved with BamHI and HindIII, and CaMV35S promoter (Benfey et al., Science, 250, 959-966(1993)) is then inserted thereto. A hygromycin-resistant gene (hygromycin phosphotransferase, hph) (Gritz et al., (1983)) is inserted at BglII site of the thus-obtained plasmid. This plasmid is cleaved with HindIII and ClaI, end-blunted, and then ligated. The thus-obtained plasmid is cleaved with SacI to remove approximately 0.5 kb fragment, and then again ligated. At the BamHI site of this plasmid, a synthetic adapter having BamHI, HindIII, XbaI, SacI, HpaI, KpnI, ClaI and BglII cleavage sites is inserted to construct a plasmid pGA1182.
The PCR is carried out by using the plasmid PGA1182 as a template, 5'-TTGGCATGCACATAC-3' (SEQ ID NO:5) as primer 1, and 5'-CGGGATCCGTGTTTGACAGG-3' (SEQ ID NO:6) as primer 2.
The approximately 120 bp fragment obtained is cleaved with BamHI and SphI, and then ligated into an approximate 9 kb fragment, which is obtained by cleaving the plasmid pGA1182 with the same restriction enzymes. Between BamHI and ClaI sites of the thus-obtained plasmid, the GUS gene (Jefferson et al., EMBO J, 6, 3901-3907(1987)) is inserted to construct the binary vector pGA1633.
pGA1173-9 containing 2.9 kb RA8 genomic DNA as constructed in step 3 of Example 1 is subjected to PCR with synthetic oligomer primers of SEQ ID NO:8 (AATTAACCCTCACTAAAGGG) and SEQ ID NO:9 (GCGGATCCAGGTTGAACCAC). The synthetic oligomer shown in SEQ ID NO:9 generates a BamHI site in exon 2 of the RA8 gene.
Then plasmid pGA1639, containing the amplified sequence as above, is constructed. The plasmid pGA1639 contains the 2.7 kb SacI-BamHI fragment comprising the 5' flanking region, exon 1, intron 1, and a part of exon 2 of the RA8 gene.
This fragment is connected to the beta-glucuronidase coding sequence in the pGA1633, to construct the plant expression vector pGA1647. In the vector pGA1647, the above described RA8 gene-derived sequence is fused at the newly introduced BamHI site within exon 2 with the GUS gene without any changes of the reading frame. Thus, a translational fusion is made between part of the RA8 gene and the beta-glucuronidase coding sequence.

### Example 3: Production of the transgenic rice

The expression vector pGA1647 constructed in Example 2, is transformed into *Agrobacterium tumefaciens* LBA4404 (Hoekema et al., Nature, 303, 179-181(1983)) carrying the binary Ti plasmid pAL4404 using the freeze-thaw method (An, G. et al., (eds) Plant Molecular Biology Manual, pp. A3/1-A3/19, Kluwer Academic Publishers, Dordrecht (1988)).
The transformed Agrobacterium tumefaciens LBA4404 is grown for 3 days in the AB liquid medium supplemented with 30 mg/L hygromycin B and 3 mg/L tetracycline, and it is co-cultivated with three-week-old calli which are induced from the scutellum of mature seeds in the N6 medium (Chu, C.C. et al., Sci, Sin., 18, 659-668(1975)) containing 2 mg/L 2,5-D, on the 2N6-As medium supplemented with 1 mM betaine (Hiei, Y. et al., Plant J., 6, 271-282(1994)) in darkness at 25 °C for 2-3 days. The co-cultivated calli are washed with sterile water containing 100 mg/L cefotaxime, and again incubated on an N6 medium containing 40 mg/L hygromycin and 250 mg/L cefotaxime for 3 weeks. Actively growing hygromycin-resistant calli are transferred onto the selection medium [for example, MS media (Life Technologies) + 0.2 mg/L NAA (naphthalene acetic acid) + 2 mg/L kinetin + 2% sorbitol + 1.6% phytagar (Gibco) + 50 mg/L hygromycin B + 250 mg/L cefotaxime], and then cultivated for 2-3 weeks under continuous light condition of 40 µmol m⁻² s⁻¹. The thus-obtained plantlets are potted and grown in a growth chamber under 10h light/14h dark condition to obtain a total of 22 transgenic rice plants.
In order to select a plant line having the highest expression level in anthers among the twenty-two (22) transgenic rice plants, histochemical GUS staining is performed for the floral portion of the transgenic rice according to the method of Dai et al. [Dai, Z. et al., Plant Mol. Biol., 32, 1055-1065 (1996)], which is modified to contain 20% methanol in the staining solution, and then fixed in a fixing solution (50% ethanol, 5% acetic acid, and 3.7% formaldehyde). The samples are observed under a dissecting microscope (x7.5 magnification). From this result, the rice exhibiting the highest GUS expression level in anthers, is selected and designated as a transgenic plant line A11279.

### Example 4: Histochemical GUS analysis of the transgenic rice

To study the spatial and temporal expression patterns of the RA8 gene, young flowers, flowers at the early vacuolated-pollen stage, mature flowers, leaves, and roots from the transgenic rice A11279 produced in Example 3, are histochemically analyzed for GUS activity as described in Example 3, and then observed under a microscope. As a control, a flower of untransformed rice is used.
GUS expression driven by the RA8 promoter is detected only in the anther of rice flowers, but not in other floral organs or in leaves or roots. In addition, the expression level of the GUS reporter gene increased as flowers matured, and the gene is not expressed at all iri the young flowers at pre-meiosis stage.
To study rice anther-specific expression patterns of the RA8 promoter-reporter gene construct, after staining and fixing four rice anthers at different developmental stages according to the same method as described in Example 3, they are embedded in a Paraplast (Sigma), and sectioned to 10 µm thickness. The sections are observed under an optical microscope (x100 magnification) using dark-field illumination. At the pre-meiosis stage, GUS activity is not detected in any part of the anther. The GUS expression is first detectable at the time when microspores are released from tetrads. The highest amount of GUS activity is observed in anthers at the vacuolated pollen stage. In anthers at mature pollen stage just before or after dehiscence, however, GUS activity suddenly decreased. It is observed that the GUS staining is restricted to tapetum, connective, and endothecium tissues.

### Example 5: Production of transgenic maize

Transformation of maize with a novel gene prepared according to the above method is achieved by microprojectile bombardment of either immature zygotic embryos or serially-propagatable Type I embryogenic callus.
Type I embryogenic maize callus cultures (Green *et al*, Miami Winter Symposium 20,1983) are initiated from immature embryos, 1.5 - 2.5 mm in length, from greenhouse grown material. Embryos are aseptically excised from surface-sterilized ears approximately 14 days after pollination. Embryos may be placed on D callus initiation media with 2% sucrose and 5 mg/L chloramben (Duncan *et al*, Planta 165: 322-332,1985) or onto KM callus initiation media with 3% sucrose and 0.75 mg/L 2,4-d (Kao and Michayluk, Planta 126:105-110, 1975). Embryos and embryogenic cultures are subsequently cultured in the dark. Embryogenic responses are removed from the explants after ~14 days. Embryogenic responses from D callus initiation media are placed onto D callus maintenance media with 2% sucrose and 0.5 mg/L 2,4-d while those from KM callus initiation media are placed onto KM callus maintenance media with 2% sucrose and 5 mg/L Dicamba. After 3 to 8 weeks of weekly selective subculture to fresh maintenance media, high quality compact embryogenic cultures are established. Actively growing embryogenic callus pieces are selected as target tissue for gene delivery. The callus pieces are plated onto target plates containing maintenance medium with 12% sucrose approximately 4 hours prior to gene delivery. The callus pieces are arranged in circles, with radii of 8 and 10 mm from the center of the target plate.
Plasmid DNA is precipitated onto gold microcarriers as described in the DuPont Biolistics manual. Two to three µg of each plasmid is used in each 6 shot microcarrier preparation.
Genes are delivered to the target tissue cells using the PDS-1000He Biolistics device. The settings on the Biolistics device are as follows: 8 mm between the rupture disc and the macrocarrier, 10 mm between the macrocarrier and the stopping screen and 7 cm between the stopping screen and the target. Each target plate is shot twice using 650 psi rupture discs. A 200 X 200 stainless steel mesh (McMaster-Carr, New Brunswick, NJ) is placed between the stopping screen and the target tissue.
Seven days after gene delivery, target tissue pieces are transferred from the high osmotic medium to selection media. For selection using the BAR gene, target tissue pieces are placed onto maintenance medium containing 100 mg/L glufosinate ammonium (Basta®) or 20 mg/L bialaphos (Herbiace®). All amino acids are removed from the selection media. After 5 to 8 weeks on these high level selection media, any growing callus is subcultured to media containing 3-20 mg/L Basta®.
For selection using the Mannose Phosphate Isomerase gene, target tissues are placed on their respective maintenance media containing no sucrose and 1% mannose. The amino acids are not removed from these media. After 5 to 8 weeks, growing callus is either
subcultured to D callus maintenance medium containing no sucrose and 1.5% mannose or KM callus maintenance medium containing 1% sucrose and 0.5% mannose. Embryogenic callus growing on selection media is subcultured every 2 weeks for 4 to 8 weeks until enough callus is produced to generate 10-20 plants. Tissue surviving selection from an original target tissue piece is subcultured as a single colony and designated as an independent transformation event.
At that point, colonies selected on Basta® are transferred to a modified MS medium (Murashige and Skoog, Physiol. Plant, 15:473-497, 1962) containing 3% sucrose (MS3S) with no selection agent and placed in the light. Either 0.25 mg/L ancymidol and 0.5 mg/L kinetin are added to this medium to induce embryo germination or 2 mg/L benzyl adenine is added. Colonies selected using mannose are transferred onto a modified MS medium containing 2% sucrose and 1% mannose (MS2S + 1M) with the ancymidol and kinetin additions described above or a modified MS medium containing 2% sucrose and 0.5% mannose (MS2S + 0.5M) with the benzyl adenine addition described above.
Regenerating colonies from Basta® selection are transferred to MS3S media without ancymidol and kinetin or benzyl adenine after 2 weeks. Regenerating colonies from mannose selection are transferred to MS2S + 1 M and MS2S + 0.5M media respectively without hormones after 2 weeks. Regenerating shoots with or without roots from all colonies are transferred to Magenta boxes containing MS3S medium and small plants with roots are eventually recovered and transferred to soil in the greenhouse.
Plants are tested for expression of the PMI gene using a modified 48-well chlorophenol red assay where the media contains no sucrose and 0.5% mannose. Leaf samples (-5 mm x 5 mm) are placed on this assay media and grown in the dark for -72 hours. If the plant is expressing the PMI gene, it can metabolize the mannose and the media will turn yellow. If not, the media will remain red.
Transformation events have also been created using Type I callus obtained from immature zygotic embryos using standard culture techniques. For gene delivery, approximately 300 mg of the Type I callus is prepared by subculturing to fresh media 1 to 2 days prior to gene delivery, selecting target tissue pieces and placing them in a ring pattern 10 mm from the center of the target plate on medium again containing 12% sucrose. After approximately 4 hours, the tissue is bombarded using the PDS-1000/He Biolistic device from DuPont. Plasmids of interest are precipitated onto 1 µm gold particles using the standard protocol from DuPont. Genes are delivered using two shots per target plate at 650 psi. Approximately 16 hours after gene delivery the callus is transferred to standard culture medium containing 2% sucrose with no selection agent. At 12 or 13 days after gene delivery, target tissue pieces are transferred to selection media containing 40 mg/l phosphinothricin as either Basta or bialaphos. The callus is subcultured on selection for 12 to 16 weeks, after which surviving and growing callus is transferred to standard regeneration medium containing 3 mg/l phosphinothricin as Basta for the production of plants.

### Example 6: Transformation of wheat

A preferred technique for wheat transformation involves particle bombardment of immature wheat embryos and includes either a high sucrose or a high maltose step prior to gene delivery. Prior to bombardment, any number of embryos (0.75-1 mm in length) are plated onto MS medium with 3% sucrose (Murashige and Skoog, 1962) and 3 mg/l 2,4-D for induction of somatic embryos which is allowed to proceed in the dark. On the chosen day of bombardment, embryos are removed from the induction medium and placed onto the osmoticum (*i*.*e*. induction medium with sucrose or maltose added at the desired concentration, typically 15%). The embryos are allowed to plasmolyze for 2-3 h and are then bombarded. Twenty embryos per target plate is typical, although not critical. An appropriate gene-carrying plasmid is precipitated onto micrometer size gold particles using standard procedures. Each plate of embryos is shot with the DuPont Biolistics helium device using a burst pressure of ~1000 psi and using a standard 80 mesh screen. After bombardment, the embryos are placed back into the dark to recover for about 24 h (still on osmoticum). After 24 hrs, the embryos are removed from the osmoticum and placed back onto induction medium where they stay for about a month before regeneration. Approximately one month later the embryo explants with developing embryogenic callus are transferred to regeneration medium (MS + 1 mg/liter NAA, 5 mg/liter GA), further containing the appropriate selection agent. After about one month, developed shoots are transferred to larger sterile containers known as GA7s which contained half-strength MS, 2% sucrose, and the same concentration of selection agent. The stable transformation of wheat is described in detail in patent application EP 0 674 715.

## Claims

1. A DNA molecule comprising a promoter sequence and an associated coding sequence wherein the promoter sequence drives expression of the coding sequence specifically in the tapetum, endothecium and connective tissues of anthers but not in microspores or pollen, and wherein expression of the coding sequence starts at the tetrad stage and reaches a maximum level at the vacuolated pollen stage wherein said promoter sequence comprises a nucleotide sequence having 50 % or more sequence identity with the sequence shown in SEQ ID NO:3.

2. A DNA molecule according to claim 1, wherein the promoter sequence comprises the nucleotide sequence shown in SEQ ID NO:3.

3. A DNA molecule according to claim 1 or claim 2, wherein the coding sequence is in antisense orientation.

4. A DNA molecule according to any one of claims 1 to 3, wherein the coding sequence encodes a polypeptide which will disrupt formation of viable pollen when expressed in the anther cells.

5. A DNA molecule according to claim 4, wherein the coding sequence encodes a polypeptide selected from the group consisting of RNase, DTA, TURF-13, pectate lyase, gin recombinase, iaaL and cytA toxin.

6. A DNA molecule according to any one of claims 1 to 5, wherein the promoter sequence comprises an additional sequence that can be operatively linked to a coding sequence of interest wherein the additional sequence comprises a sequence having 50 % or more sequence identity with SEQ ID NO:10.

7. A DNA molecule according to claim 6, wherein the additional sequence comprises SEQ ID NO:10.

8. A DNA molecule according to claim 6 or claim 7, wherein the promoter sequence and additional sequence have 50 % or more sequence identity with the respective sequences of SEQ ID NO:2.

9. A DNA molecule according to claim 8, wherein the promoter sequence and additional sequence are **characterized by** the nucleotide sequence of SEQ ID NO:2.

10. An expression vector comprising a first expression cassette comprising a DNA of any one of claims 1 to 9 for expression in a host organism such as a microorganism or a plant and, optionally, a second expression cassette comprising a gene of interest.

11. A transgenic plant and the sexual and/or asexual progeny thereof, which has been transformed with a DNA sequence according to any one of claims 1 to 9.

12. A transgenic, male-sterile plant which has been transformed with a DNA sequence according to any one of claims 1 to 9.

13. A transgenic plant according to any one of claims 11 or 12, wherein the plant is rice, wheat, maize, Sorghum bicolor or orchardgrass.

14. A process for the production of a transgenic plant cell containing a DNA comprising a promoter sequence and associated coding sequence wherein the promoter sequence drives expression of the coding sequence specifically in the tapetum, endothecium and connective tissues of anthers but not in microspores or pollen, and wherein expression of the coding sequence starts at the tetrad stage and reaches a maximum level at the vacuolated pollen stage said process comprising introducing a DNA according to any one of claims 1 to 9 into a plant cell.

15. A process according to claim 14, wherein the plant cell is rice, wheat, maize, Sorghum bicolor or orchardgrass cell.

## Revendications

1. Molécule d'ADN comprenant une séquence promoteur et une séquence de codage associée, dont la séquence promoteur commande l'expression de la séquence de codage spécifiquement dans les tissus du tapetum, de l'endothécium et du connectif des anthères, mais pas dans les microspores ni le pollen, et pour laquelle l'expression de la séquence de codage commence au stade tétrade et atteint un niveau maximum au stade pollen vacuolé, ladite séquence promoteur comprenant une séquence nucléotidique ayant 50 % d'identité de séquence ou plus avec la séquence représentée dans SEQ ID n°: 3.

2. Molécule d'ADN selon la revendication 1, dans laquelle la séquence promoteur comprend la séquence nucléotidique représentée dans SEQ ID n°: 3.

3. Molécule d'ADN selon la revendication 1 ou 2, dans laquelle la séquence de codage est dans une orientation anti-sens.

4. Molécule d'ADN selon l'une quelconque des revendications 1 à 3, dans laquelle la séquence de codage code pour un polypeptide qui perturbera la formation de pollen viable quand la séquence est exprimée dans les cellules des anthères.

5. Molécule d'ADN selon la revendication 4, dans laquelle la séquence de codage code pour un polypeptide choisi dans le groupe constitué par la ribonucléase, le DTA, le TURF-13, la pectate lyase, la recombinase gin, l'iaaL et la toxine cytA.

6. Molécule d'ADN selon l'une quelconque des revendications 1 à 5, dans laquelle la séquence promoteur comprend une séquence supplémentaire qui peut être liée de façon opérationnelle à une séquence de codage intéressante, la séquence supplémentaire comprenant une séquence ayant 50 % d'identité de séquence ou plus avec SEQ ID n°: 10.

7. Molécule d'ADN selon la revendication 6, dans laquelle la séquence supplémentaire comprend SEQ ID n°: 10.

8. Molécule d'ADN selon la revendication 6 ou la revendication 7, dans laquelle la séquence promoteur et la séquence supplémentaire ont 50 % d'identité de séquence ou plus avec les séquences respectives de SEQ ID n°: 2.

9. Molécule d'ADN selon la revendication 8, dans laquelle la séquence promoteur et la séquence supplémentaire sont **caractérisées par** la séquence nucléotidique de SEQ ID n°: 2.

10. Vecteur d'expression comprenant une première cassette d'expression comprenant un ADN selon l'une quelconque des revendications 1 à 9 pour l'expression dans un organisme hôte tel qu'un microorganisme ou une plante et, de façon optionnelle, une seconde cassette d'expression comprenant un gène intéressant.

11. Plante transgénique, et sa descendance par multiplication sexuée et/ou asexuée, qui a été transformée par une séquence d'ADN selon l'une quelconque des revendications 1 à 9.

12. Plante transgénique, mâle-stérile, qui a été transformée par une séquence d'ADN selon l'une quelconque des revendications 1 à 9.

13. Plante transgénique selon l'une quelconque des revendications 11 ou 12, la plante étant le riz, le blé, le maïs, le sorgho bicolore ou le dactyle.

14. Procédé pour la production d'une cellule de plante transgénique contenant un ADN comprenant une séquence promoteur et une séquence de codage associée, où la séquence promoteur commande l'expression de la séquence de codage spécifiquement dans les tissus du tapetum, de l'endothécium et du connectif des anthères, mais pas dans les microspores ni le pollen, et où l'expression de la séquence de codage commence au stade tétrade et atteint un niveau maximum au stade pollen vacuolé, ledit procédé comprenant l'introduction d'un ADN selon l'une quelconque des revendications 1 à 9 dans une cellule de plante.

15. Procédé selon la revendication 14, où la cellule de plante est une cellule de riz, de blé, de maïs, de sorgho bicolore ou de dactyle.

## Patentansprüche

1. DNA-Molekül, das eine Promotorsequenz und eine assoziierte codierende Sequenz umfaßt, worin die Promotorsequenz die Expression der codierenden Sequenz spezifisch im Tapetum, Endothezium und Bindegeweben von Staubbeuteln antreibt, aber nicht in Mikrosporen oder Pollen, und worin die Expression der codierenden Sequenz im Tetradenstadium beginnt und eine maximale Stärke im vakuolisierten Pollenstadium erreicht, worin die Promotorsequenz eine Nukleotidsequenz mit 50 % oder mehr Sequenzidentität mit der in SEQ ID NO: 3 gezeigten Sequenz umfaßt.

2. DNA-Molekül gemäß Anspruch 1, worin die Promotorsequenz die in SEQ ID NO: 3 gezeigte Nukleotidsequenz umfaßt.

3. DNA-Molekül gemäß Anspruch 1 oder 2, worin die codierende Sequenz in antisense-Orientierung ist.

4. DNA-Molekül gemäß einem der Ansprüche 1 bis 3, worin die codierende Sequenz ein Polypeptid codiert, das bei Expression in den Staubbeutelzellen die Bildung von lebensfähigen Pollen unterbrechen wird.

5. DNA-Molekül gemäß Anspruch 4, worin die codierende Sequenz ein Polypeptid codiert, das aus der Gruppe ausgewählt ist, die aus RNase, DTA, TURF-13, Pectatlyase, Ginrekombinase, iaaL und cytA-Toxin besteht.

6. DNA-Molekül gemäß einem der Ansprüche 1 bis 5, worin die Promotorsequenz eine zusätzliche Sequenz umfaßt, die funktionsfähig mit einer codierenden Sequenz von Interesse verbunden werden kann, worin die zusätzliche Sequenz eine Sequenz mit 50 % oder mehr Sequenzidentität mit SEQ ID NO: 10 umfaßt.

7. DNA-Molekül gemäß Anspruch 6, worin die zusätzliche Sequenz SEQ ID NO: 10 umfaßt.

8. DNA-Molekül gemäß Anspruch 6 oder 7, worin die Promotorsequenz und die zusätzliche Sequenz 50 % oder mehr Sequenzidentität mit den jeweiligen Sequenzen von SEQ ID NO: 2 haben.

9. DNA-Molekül gemäß Anspruch 8, worin die Promotorsequenz und die zusätzliche Sequenz durch die Nukleotidsequenz von SEQ ID NO: 2 **gekennzeichnet** sind.

10. Expressionsvektor, der eine erste Expressionskassette, die eine DNA gemäß einem der Ansprüche 1 bis 9 zur Expression in einem Wirtsorganismus wie einem Mikroorganismus oder einer Pflanze umfaßt, und gegebenenfalls eine zweite Expressionskassette, die ein interessierendes Gen umfaßt, umfaßt.

11. Transgene Pflanze und geschlechtliche und/oder ungeschlechtliche Nachkommenschaft davon, die mit einer DNA-Sequenz gemäß einem der Ansprüche 1 bis 9 transformiert wurde.

12. Transgene männlich-sterile Pflanze, die mit einer DNA-Sequenz gemäß einem der Ansprüche 1 bis 9 transformiert wurde.

13. Transgene Pflanze gemäß einem der Ansprüche 11 oder 12, worin die Pflanze Reis, Weizen, Mais, Sorghum bicolor oder Knaulgras ist.

14. Verfahren zur Herstellung einer transgenen Pflanzenzelle, die eine DNA enthält, die eine Promotorsequenz und assoziierte codierende Sequenz umfaßt, worin die Promotorsequenz die Expression der codierenden Sequenz spezifisch im Tapetum, Endothezium und Bindegeweben der Staubbeutel antreibt, aber nicht in Mikrosporen oder Pollen und worin die Expression der codierenden Sequenz im Tetradenstadium beginnt und eine maximale Stärke im vakuolisierten Pollenstadium erreicht, wobei das Verfahren das Einführen einer DNA gemäß einem der Ansprüche 1 bis 9 in eine Pflanzenzelle umfaßt.

15. Verfahren gemäß Anspruch 14, worin die Pflanzenzelle Reis, Weizen, Mais, Sorghum bicolor oder Knaulgras ist.
